# EUROPEAN PATENT SPECIFICATION

(11) **EP 2 591 359 B1**
(45) Date of publication and mention of the grant of the patent: **01.03.2017**
(21) Application number: 11804372.8
(22) Date of filing: 07.07.2011
(51) Int. Cl.: G01N 33/53, C12Q 1/06, G01N 33/58

(54) **METHODS FOR QUANTIFYING EXOSOMES**
VERFAHREN ZUR QUANTIFIZIERUNG VON EXOSOMEN
PROCÉDÉS PERMETTANT DE QUANTIFIER DES EXOSOMES

(30) Priority: 07.07.2010 US 362129 P
(43) Date of publication of application: 15.05.2013
(73) Proprietor: Aethlon Medical Inc, San Diego, CA 92123 (US)
(72) Inventor: TULLIS, Richard, H., Encinitas CA 92024 (US); DUFFIN, Paul, San Diego CA 92110 (US)
(74) Representative: Plougmann Vingtoft a/s
(86) International application number: PCT/US2011/043265
(87) International publication number: WO 2012/006476

(56) References cited:
- EP-A1- 0 977 036
- WO-A1-2012/170711
- WO-A2-2007/103572
- WO-A2-2009/092386
- WO-A2-2009/092386
- WO-A2-2010/056337
- WO-A2-2010/065765
- WO-A2-2010/065765
- WO-A2-2010/141862
- WO-A2-2010/141862
- JOURNAL OF IMMUNOTHERAPY, vol. 35, no. 9, November 2012 (2012-11), pages 778-779, XP008166223, 27TH ANNUAL SCIENTIFIC MEETING OF THE SOCIETY-FOR-IMMUNOTHERAPY-OF-CANCER (SITC); NORTH BETHESDA, MD, USA; OCTOBER 24 -28, 2012
- Juan Echevarria ET AL: "Microarray-Based Identification of Lectins for the Purification of Human Urinary Extracellular Vesicles Directly from Urine Samples", ChemBioChem, vol. 15, no. 11, 8 July 2014 (2014-07-08), pages 1621-1626, XP055185626, ISSN: 1439-4227, DOI: 10.1002/cbic.201402058
- Alla Kachko ET AL: "Inhibition of Hepatitis C Virus by the Cyanobacterial Protein Microcystis viridis Lectin: Mechanistic Differences between the High-Mannose Specific Lectins MVL, CV-N, and GNA", Molecular Pharmaceutics, vol. 10, no. 12, 2 December 2013 (2013-12-02), pages 4590-4602, XP055262967, US ISSN: 1543-8384, DOI: 10.1021/mp400399b

## Description

### RELATED APPLICATIONS

### FIELD OF THE INVENTION

Embodiments of the present invention relate to methods for quantifying exosomes.

In particular, methods that utilize lectins to quantify exosomes are provided.

### BACKGROUND OF THE INVENTION

Exosomes include small vesicles released by mammalian cells for a number of purposes including immunomodulation. Depending on the conditions, exosomes can either be immunostimulatory or immunosuppressive. During pregnancy, exosomes inhibit the production of certain T-cells thereby protecting the fetus (Taylor, D.D., et al., Pregnancy-associated exosomes and their modulation of T cell signaling. J Immunol, 2006. 176(3): 1534-42). In the case of certain bacterial infections, exosomes derived from infected cells express antigenic fragments of the bacterium to stimulate the immune system against the pathogen (Bhatnagar, S., et al., Exosomes released from macrophages infected with intracellular pathogens stimulate a proinflammatory response in vitro and in vivo. Blood, 2007. 110(9): 3234-44). It has been postulated that cancers utilize the immunomodulatory properties of exosomes in order to evade the immune system (Taylor D.D. et al., (2005) Tumor-derived exosomes and their role in cancer associated T-cell signaling defects. Brit. J. of Cancer 92:305-311).

While there is increasing evidence of the importance of exosomes in the progression and prognosis of cancers and infectious diseases both as a means of treatment and as a diagnostic, there is not currently an assay that reliably detects exosomes from a number of cell types. Exosomes are currently characterized and purified by methods such as size chromatography and general protein assay. Exosomes may also be purified using antibodies specific to particular exosome epitopes (*See*, *e.g.,* U.S. Pat. App. No. 20090220944). However, methods using such antibodies are limited to exosomes that display particular antigens only. In view of the increasing importance of exosomes in the diagnosis and prognosis of certain diseases, there is an need for simple and efficient methods to quantify exosomes that is generally applicable to exosomes from a number of cell types. WO2010056337 discloses a method to determine the bio-signature of exosomes in a sample, wherein the determination of the bio-signature may be the quantification of isolated exosomes. The method comprises capturing exosomes using a binding agent, such as a lectin.

### SUMMARY OF INVENTION

In its broadest sense the invention is defined by the scope of the appended claims covering the following aspects:
1. A method for quantifying exosomes in a sample comprising: contacting the sample with lectin immobilized on a substrate; contacting exosomes bound to the lectin with a detectable exosome-binding agent; and measuring a signal from the bound detectable exosome-binding agent, thereby quantifying the bound exosomes; wherein the lectin is selected from the group consisting of *Galanthus nivalis* lectin (GNA), *Narcissus pseudonarcissus* lectin (NPA), *Allium sativum* lectin (ASA), *Lens culinaris* lectin (LCH), *Sambucus nigra* lectin (SNA), *Maackia amurensis* lectin (MAL), and concanavalin A.
2. The method of 1, further comprising comparing the signal from the bound detectable binding agent to a signal on a standard curve.
3. The method of any one of 1-2, wherein the exosomes are derived from a cell selected from the group consisting of an ovarian cancer cell, a melanoma cell, a colon cancer cell, and a tuberculosis infected cell.
4. The method of any one of 1-3, wherein the method provides a sensitivity of detecting exosomes in the sample that is at least 1 x 10⁹ exosomes/ml, preferably at least 1 x 10⁸ exosomes/ml, or more preferably at least 1 x 10⁷ exosomes/ml.
5. The method of any one of 1-4, wherein the sample comprises an exosome having a diameter of 10 nm to 800 nm, or preferably a diameter of 30 nm to 200 nm.
6. The method of any one of 1-5, wherein the sample comprises exosomes isolated from a fluid by a method selected from the group consisting of size exclusion chromatography, density gradient centrifugation, differential centrifugation, nanomembrane ultrafiltration, immunoabsorbent capture, affinity purification, microfluidic separation, and a combination thereof.
7. The method of any one of 1-6, wherein at least one of the lectin immobilized on the substrate and the detectable binding agent are capable of binding to exosomes derived from a plurality of cell types.
8. The method of any one of 1-7, wherein the detectable exosome-binding agent is selected from the group consisting of an antibody, an antibody fragment, and a lectin selected from the group consisting of *Galanthus nivalis* lectin (GNA), *Narcissus pseudonarcissus* lectin (NPA), *Allium sativum* lectin (ASA), *Lens culinaris* lectin (LCH), *Sambucus nigra* lectin (SNA), *Maackia amurensis* lectin (MAL), and concanavalin A; and optionally wherein the detectable exosome-binding agent comprises a detectable label selected from the group consisting of an enzyme, a chemiluminescent agent, a fluorescent agent, and an isotope.
9. The method of any one of 1-8, wherein the sample is selected from the group consisting of peripheral blood, serum, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, umbilical cord blood, and ascites fluid.
10. The method of any one of 1-9, wherein the substrate comprises a multiwell plate comprising a material selected from the group consisting of sepharose, latex, glass, polystyrene, polyvinyl, nitrocellulose and silicon.

### BRIEF DESCRIPTION OF DRAWINGS

FIG. 1 is a plot of absorbance at 450 nm versus concentration of mannan measured by an embodiment of an assay of the present invention.
FIG. 2 is a plot of absorbance at 450 nm versus concentration of mannan coated latex beads measured by an embodiment of an assay of the present invention.
FIG. 3 depicts a mannan standard curve and a purified exosome preparation measured by an embodiment of an assay of the present invention.
FIG. 4 depicts a dilution of an exosome sample measured by an embodiment of an assay of the present invention.

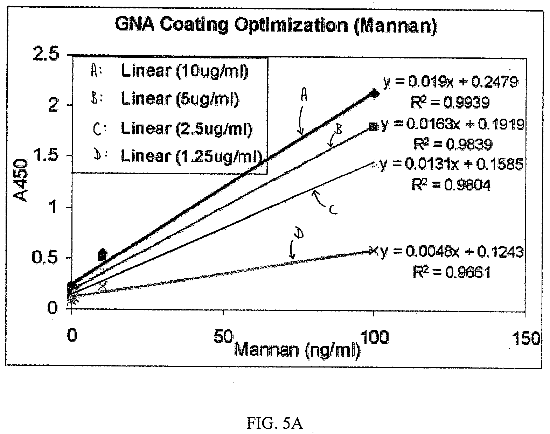

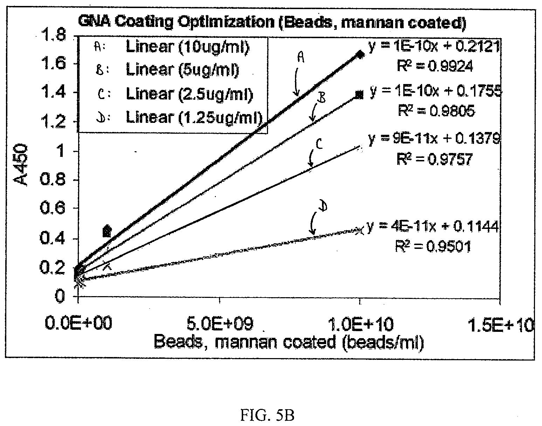

FIGs. 5A and 5B depict a mannan (FIG. 5A) and mannan coated latex bead (FIG. 5B) optimization experiment where known quantities of mannan and mannan beads were incubated in 96 well plate wells that were coated with different amounts of GNA (10 µg/ml, 5 µg/ml, 2.5 µg/ml, and 1.25 µg/ml).
FIG. 6 depicts a mannan bead standard curve measured by an embodiment of an assay of the present invention.
FIG. 7 depicts a graph of quantities of exosomes present in a sucrose-gradient purified sample or a GNA column purified sample, relative to a mannan standard curve measured by an embodiment of an assay of the present invention.
FIG. 8 depicts a graph of ovarian cancer exosomes detected using an anti-PLAP antibody in an embodiment of an assay of the present invention.

### DETAILED DESCRIPTION

Embodiments of the present invention relate to methods that utilize lectins to quantify exosomes. Lectins such as concanavalin A and *Galanthus Nivalis* lectin (GNA) bind specifically to cancer cells indicating that these cells contain sugar moieties uncommon to healthy cells. Cancer specific exosomes secreted from these cells have the same moieties, and are therefore be available to lectin capture and detection.

Assays such as the enzyme linked immunosorbent assay (ELISA) is a powerful tool that has been used for the past 4 decades to detect proteins in homogenous and heterogenous samples, generally by adsorbing an antigen specific antibody to a solid surface such as a 96 well plastic plate, incubating the antigen with the adsorbed antibody, and detecting the antigen with a secondary antibody labeled with various chemicals that react to give color, fluorescence or other means of detection.

However, the use of ELISA for the detection of exosomes requires an antibody which is specific for a surface antigen on the exosome which is not present on normal cells. The development of antibodies for use with an ELISA assay can be difficult and tedious. Therefore, there is a need for methods and compositions that specifically and reliably capture and/or detect exosomes, preferably in a quantitative manner.

Beyond the potential therapeutic benefits of eliminating immunosuppressive exosomes from circulation, researchers recognize that exosomes represent an important diagnostic target to determine progression and prognosis of both cancers and infectious disease conditions. However, the availability of functional assays that specifically detect exosomes is limited. At present, exosomes are generally characterized and purified by size chromatography and general protein assay, not by chemical structures specific to the exosome. Some embodiments of the present technology include Enzyme Linked Lectin Specific Assays (ELLSA) designed to bind specifically to carbohydrate structures common to exosomes, but not to healthy human cellular components. In some such embodiments, each ELLSA plate allows for up to 96 exosome detection tests. Further analysis of the captured exosomes is possible through detection molecules such as antibodies linked to a specific biomarker on the exosome.

The Hemopurifier® is a medical device that selectively targets the removal of infectious viruses and immunosuppressive proteins from the entire circulatory system (*See, e.g.,* U.S. Pat. No. 7226429). It has been discovered that devices such as the Hemopurifier® capture tumor-secreted exosomes that suppress the immune system of those afflicted with cancer (*See e.g.,* U.S. Pat. App. No. 20090304677). Prior to this discovery, a therapeutic strategy to directly inhibit or reverse the immunosuppressive destruction caused by exosomes did not exist in cancer care. By eliminating this mechanism, the Hemopurifier® can fill an unmet clinical need and provide the benefit of an immune-based therapy without adding drug toxicity or interaction risks to established and emerging treatment strategies

In some embodiments, the Hemopurifier® is a selective filtration device containing affinity agents that tightly bind to high-mannose structures unique to the surface of exosomes produced by cancer and glycoproteins residing on the envelope of viruses. Agents can include some biomarkers (*See, e.g.,* Int. Pat. Pub. No. WO 2010/065765). These agents are immobilized around approximately 2800 porous hollow fibers that run the interior length of the device. The resulting design enhances the ability to separate both exosome and viral targets away from blood cells so they can then be selectively and permanently removed from the circulatory system. In some applications, blood circulation is established into the Hemopurifier® via a catheter or other blood access device. Once blood flow has been established, treatment benefit is immediate as the entire circulatory system can pass through the Hemopurifier®, in some embodiments, in as little as 15 minutes. However, there remains a need to measure the concentration of exosomes in patient samples to gauge the effectiveness of the Hemopurifier® treatment. Embodiments of the present invention satisfy this need by providing methods for quantifying a broad spectrum of exosomes in samples.

### Biological samples

Some embodiments of the methods and compositions provided herein include a biological sample. Biological samples include, for example, cell culture media, and samples obtained from a subject. As used herein, the term "subject" includes an animal, a mammal, and a human. A sample obtained from a subject can include any tissue or fluid from the subject that may contain exosomes. Examples of biological samples obtained from a subject that may contain exosomes include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids. A biological sample may also include the blastocyl cavity, umbilical cord blood, or maternal circulation which may be of fetal or maternal origin. The biological sample may also be a tissue sample or biopsy, from which exosomes may be obtained. For example, if the sample is a solid sample, cells from the sample can be cultured and exosome product induced. In some embodiments, the sample is ascites fluid from a subject, e.g., ascites fluid from a human subject with ovarian cancer; cell culture media supernatant from a human primary melanoma cell line; cell culture media supernatant from a human primary colon cancer cell line; or murine macrophage, e.g., murine macrophage infected with tuberculosis.

### Exosomes

Some embodiments of the methods and compositions provided herein include a sample comprising exosomes. Generally, exosomes are small vesicles that are released into the extracellular environment from a variety of different cells, for example, cells that originate from, or are derived from, the ectoderm, endoderm, or mesoderm including any such cells that have undergone genetic, environmental, and/or any other variations or alterations. An exosome is typically created intracellularly when a segment of the cell membrane spontaneously invaginates and is ultimately exocytosed (*see e.g.,* Keller et al. (2006), Immunol. Lett. 107: 102-8). In some embodiements, the exosomes have a diameter of greater than about 10 nm, 20 nm, or 30 nm; a diameter that is, or is about, 30-1000 nm, 30-800 nm, 30-200 nm, or 30-100 nm. In some embodiments, exosomes have a diameter of less than, or less than about, 10,000 nm, 1000 nm, 800 nm, 500 nm, 200 nm, 100 nm or 50 nm. Exosomes may also be referred to as microvesicles, nanovesicles, vesicles, dexosomes, bleb, blebby, prostasomes, microparticles, intralumenal vesicles, endosomal-like vesicles or exocytosed vehicles. Exosomes can also include any shed membrane bound particle that is derived from either the plasma membrane or an internal membrane. Exosomes can also include cell-derived structures bounded by a lipid bilayer membrane arising from both herniated evagination separation and sealing of portions of the plasma membrane or from the export of any intracellular membrane-bounded vesicular structure containing various membrane-associated proteins of tumor origin, including surface-bound molecules derived from the host circulation that bind selectively to the tumor-derived proteins together with molecules contained in the exosome lumen including tumor-derived microRNAs or intracellular proteins. Exosomes can also include membrane fragments.

In some embodiments, the exosomes are cancer exosomes. Cancer exosomes include exosomes from cancer cells and/or tumor cells (primary or cell culture) from cancers such as breast cancer, ovarian cancer, lung cancer, colon cancer, hyperplastic polyp, adenoma, colorectal cancer, high grade dysplasia, low grade dysplasia, prostatic hyperplasia, prostate cancer, melanoma, pancreatic cancer, brain cancer (such as a glioblastoma), hematological malignancy, hepatocellular carcinoma, cervical cancer, endometrial cancer, head and neck cancer, esophageal cancer, gastrointestinal stromal tumor (GIST), renal cell carcinoma (RCC) or gastric cancer. The colorectal cancer includes CRC Dukes B or Dukes C-D. The hematological malignancy includes B-Cell Chronic Lymphocytic Leukemia, B-Cell Lymphoma-DLBCL, B--Cell Lymphoma-DLBCL-germinal center-like, B-Cell Lymphoma-DLBCL-activated B-cell-like, and Burkitt's lymphoma. Cancer exosomes may also be derives from a premalignant condition, for example, but not limited to, Barrett's Esophagus. In some embodiments, cancer exosomes include exosomes from ovarian cancer, colon cancer, and melanoma.

### Isolation of exosomes

Some embodiments of the methods and compositions provided herein include isolating exosomes from a sample. As used herein, the term "isolating" refers to increasing the concentration or density of exosomes in a sample and, or, removing non-exosome substances (e.g., proteins, cells) from a sample. Methods of isolating exosomes are well known in the art. Examples of methods useful to isolate exosomes include but are not limited to size exclusion chromatography, density gradient centrifugation, differential centrifugation, nanomembrane ultrafiltration, immunoabsorbent capture, affinity purification, microfluidic separation, or combinations thereof. Size exclusion chromatography, such as gel permeation columns, centrifugation or density gradient centrifugation, and filtration methods can be used. For example, exosomes can be isolated by differential centrifugation, anion exchange and/or gel permeation chromatography (e.g., U.S. Pat. Nos. 6,899,863 and 6,812,023), sucrose density gradients, organelle electrophoresis (*e.g.,* U.S. Pat. No. 7,198,923), magnetic activated cell sorting (MACS), or with a nanomembrane ultrafiltration concentrator. Various combinations of isolation or concentration methods can be used.

### Substrates

Some embodiments of the methods and compositions provided herein include a substrate. In some embodiments the substrate can comprise a surface. Substrates can include but are not limited to, for example, plates, beads, and fibers. In some embodiments, a substrate comprises a multiwell plate, such as a standard 96-well plate. A substrate can comprise any suitable materials. Examples of suitable materials include but are not limited to sepharose, latex, glass, polystyrene, polyvinyl, nitrocellulose and silicon.

### Exosome-binding agents

Lectins used for this invention are selected from the group consisting of *Galanthus nivalis* lectin (GNA), *Narcissus pseudonarcissus* lectin (NPA), *Allium sativum* lectin (ASA), *Lens culinaris* lectin (LCH), *Sambucus nigra* lectin (SNA), *Maackia amurensis* lectin (MAL), and concanavalin A. In some embodiments, the lectin is GNA, NPA, SNA, or MAL. In particular embodiments, the lectin is GNA.

### Detectable moieties

Some embodiments of the methods and compositions provided herein include an exosome-binding agent or a secondary binding agent comprising a detectable moiety. Examples of detectable moieties include but are not limited to enzymes, such as horseradish peroxidase (HRP), alkaline phosphatase (AP), β-galactosidase and urease. A horseradish-peroxidase detection system can be used, for example, with the chromogenic substrate tetramethylbenzidine (TMB), which yields a soluble product in the presence of hydrogen peroxide that is detectable at 450 nm. Other convenient enzyme-linked systems include, for example, the alkaline phosphatase detection system, which can be used with the chromogenic substrate p-nitrophenyl phosphate to yield a soluble product readily detectable at 405 nm. Similarly, a β-galactosidase detection system can be used with the chromogenic substrate o-nitrophenyl-β-D-galactopyranoside (ONPG) to yield a soluble product detectable at 410 nm, or a urease detection system can be used with a substrate such as urea-bromocresol purple (Sigma Immunochemicals, St. Louis, Mo.).

More examples of detectable moieties include but are not limited to chemiluminescent labels. Methods of detecting chemiluminescent labels are known in the art. Fluorescent detection also can be useful in certain methods provided herein. Useful fluorochromes include but are not limited to, DAPI, fluorescein, Hoechst 33258, R-phycocyanin, B-phycoerythrin, R-phycoerythrin, rhodamine, Texas red and lissamine. Fluorescein or rhodamine labeled antibodies, or fluorescein- or rhodamine-labeled secondary antibodies can be useful with embodiments provided herein. An example of a secondary antibody includes an anti-GNA antibody. Isotopes can also be useful in certain methods provided herein. Such moieties and assays are well known in the art.

A signal from a detectable moiety can be analyzed, for example, using a spectrophotometer to detect color from a chromogenic substrate; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of a biomarker can be performed using a spectrophotometer such as an EMAX Microplate Reader (Molecular Devices; Menlo Park, Calif.) in accordance with the manufacturer's instructions. The assays of the invention can be automated or performed robotically, if desired, and that the signal from multiple samples can be detected simultaneously.

### Methods for quantifying exosomes

Embodiments of the methods provided herein include quantifying exosomes in a biological sample. Exosomes bound to a lectin immobilized on a substrate are detected and/or quantified using a detectable exosome-binding agent comprising a lectin, an exosome-binding antibody, or fragment thereof. In some embodiments, the detectable exosome binding agent is detected using an secondary binding agent which is labeled with a detectable moiety. For example, a secondary antibody which recognizes the detectable exosome-binding agent.

A biological sample comprising exosomes is contacted with a substrate having a surface with lectin immobilized thereon. The biological sample is incubated with the immobilized lectin, and exosomes bind to the immobilized lectin. Unbound biological sample is washed from the surface. The bound exosomes are detected and measured using a detectable exosome-binding agent, *e.g.,* an agent comprising a labeled lectin, or a labeled antibody or labeled fragment thereof. Detecting and measuring includes contacting the bound exosomes with the detectable exosome-binding agent; incubating the agent with the bound exosomes; and washing unbound agent from the exosomes bound to the lectin immobilized on the substrate. Signal from the bound detectable exosome-binding agent can be measured (optionally by contacting the exosome-binding agent with a secondary labeled binding agent) and the level of signal can be used to determine the quantity of exosomes bound to the lectin immobilized on the substrate. Methods to determine the quantity of bound exosomes using a measured signal can include comparing the level of the signal to a reference, such as a standard curve. An example standard curve includes a curve prepared using a control with various quantities of a lectin-binding compound, such as mannan or mannan coated beads. In some embodiments, the method optionally includes isolating exosomes in a sample before contacting the sample with lectin immobilized to a substrate. Methods for isolating exosomes are well known in the art and examples are also provided herein.

Exosome binding agent bound to an exosome can be detected and measured by a variety of methods. Preferably, the exosome-binding agent comprises a detectable moiety. The detectable moiety can be detected and measured using methods well known in the art; examples of such methods are provided herein. In some embodiments, the signal from the detectable moiety can be compared to a signal of a reference, for example, a standard curve. Using such well known methods, the relative quantity of bound detectable moiety can be determined. Accordingly, the relative quantity of exosomes bound by the exosome-binding moiety comprising the detectable moiety can be determined.

In some embodiments of the methods provided herein, the sensitivity of the method of detecting exosomes in a sample is, is about, is at least, or is at least about, 1 x 10¹⁰ exosomes/ml, 1 x 10⁹ exosomes/ml, 1 x 10⁸ exosomes/ml, 1 x 10⁷ exosomes/ml, or 1 x 10⁶ exosomes/ml, or a range defined by any two of the preceding values. In some embodiments of the methods provided herein, the sensitivity of detecting exosomes is relative to a reference, e.g., mannan-coated beads. In such embodiments, the sensitivity of detecting exosomes in a sample is equivalent to, is equivalent to about, is equivalent to at least, or is equivalent to at least about, 1 x 10¹⁰ beads/ml, 1 x 10⁹ beads/ml, 1 x 10⁸ beads/ml, 1 x 10⁷ beads/ml, or 1 x 10⁶ beads/ml, or a range defined by any two of the preceding values. In some embodiments, the sensitivity of detecting exosomes in the sample is, is about, is at least, or is at least about, the equivalent of 1000 ng mannan/ml, 500 ng mannan/ml, 100 ng mannan/ml, 50 ng mannan/ml, 1000 pg mannan/ml, 500 pg mannan/ml, 100 pg mannan/ml, or 50 pg mannan/ml, or a range defined by any two of the preceding values.

### EXAMPLES

### Example 1-Preparing a lectin-coated substrate

A lectin coated 96-well plate was prepared by adding 100 µl 10 µg/ml *galanthus nivalis* lectin (GNA) to each well, and incubating for 1 hour at ambient temperature. The GNA solution was removed from the wells. The immobilized lectin was blocked with 200 µl 1% BSA + 0.1 % Tween 20 in 1 x Dulbecco's phosphate buffered saline (dPBS) for 1 hour at ambient temperature.

### Example 2-Quantifying mannan bound on a lectin-coated substrate

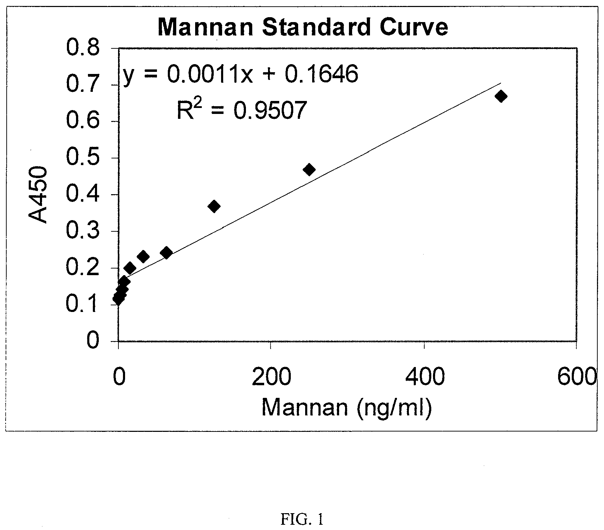

To generate a standard mannan curve, 100 µl per well of a mannan solution was incubated in each well on a plate prepared as in Example 1 for 1 hour at ambient temperature. Solutions containing100 ng/ml, 50 ng/ml, 25 ng/ml, 12.5 ng/ml, 6.25ng/ml, 3.125 ng/ml, and 1.5625 ng/ml of mannan in 1 X dPBS, and a blank with no mannan (dPBS alone), was used to create the standard curve. Mannan solution was washed off of the plate 1x with 300 µl of 0.1% Tween 20 in dPBS (wash solution) and bound mannan was detected by incubation for 1 hour at ambient temperature with 100 µl of horse radish peroxidase (HRP) labeled GNA (1 µg/ml). The plate was washed 4 x with wash solution, and detected with 100 µl tetramethylbenzadine (TMB) (SIGMA-Aldrich). TMB was incubated up to 30 minutes, or until color was seen in the blank. The TMB HRP reaction was stopped with 100 µl 1 M sulfuric acid. Resulting yellow color was measured in a plate reader at 450 nm wavelength. The resulting standard curve is shown in FIG.1.

### Example 3-Quantifying mannan beads on a lectin substrate

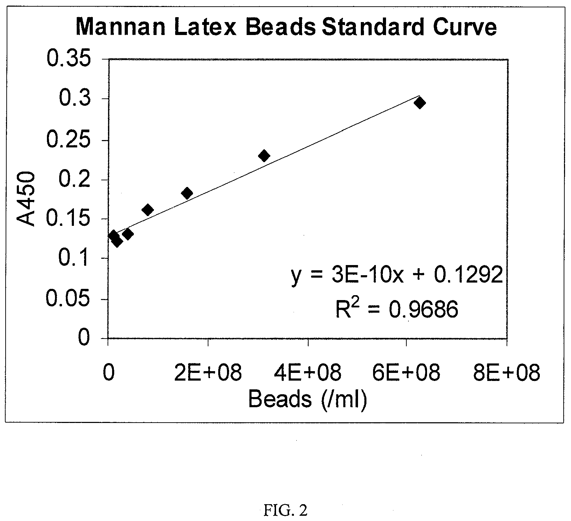

Mannan coated beads were prepared by incubating mannan at 1 mg/ml with 1e14 fluorescent latex beads (100 nm diameter), which coats the beads such that they can be captured by GNA. To prepare a standard curve, the mannan coated beads were incubated on a plate prepared as in Example 1. 100 µl of the mannan beads were incubated on the plate for 1 hour at ambient temperature at 1e10 beads/ml, 5e09 beads/ml, 2.5e09 beads/ml, 1.25e09 beads/ml, 6.25e08 beads/ml, 3.125e08 beads/ml, and 1.56e08 beads/ml, 7.81e07 beads/ml, 3.91e07 beads/ml, 1.95e07 beads/ml, 9.77e06 beads/ml in 1X dPBS, and a blank with no mannan beads (dPBS alone), to create a standard curve, part of this standard curve is shown in FIG. 2. Mannan bead solution was washed off of the plate 1 x with 300 µl of 0.1% Tween 20 in dPBS (wash solution), and bound mannan beads were detected by incubation for 1 hour at ambient temperature with 100 µl of HRP labeled GNA (1µg/ml). The plate was washed 4 x with wash solution, and detected with 100 µl of TMB. TMB was incubated up to 30 minutes, or until color was seen in the blank. The TMB HRP reaction was stopped with 1 M sulfuric acid. Resulting yellow color was measured in a plate reader at 450 nm wavelength.

### Example 4-Quantifying ovarian cancer cell exosomes

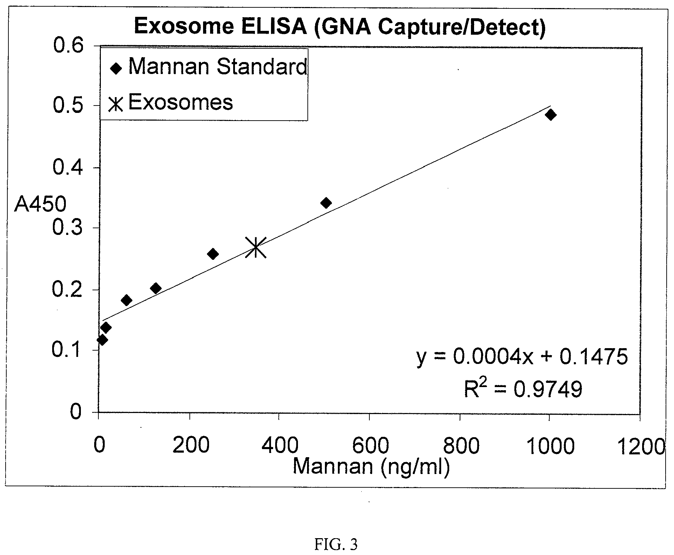

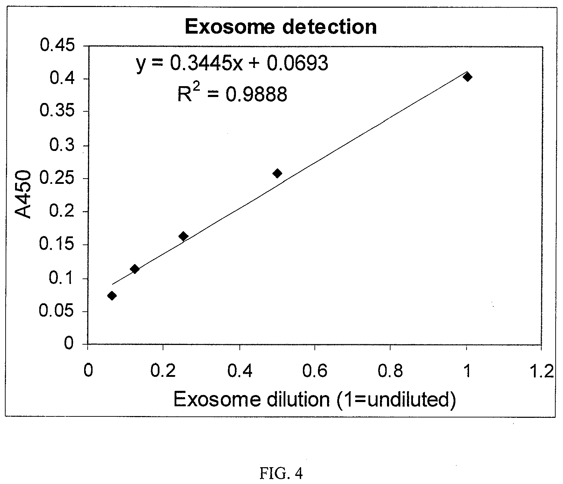

An exosome sample from ovarian cancer cells was obtained and treated according to the methods of Examples 2 and 3 using plates prepared as in Example 1. The signal generated from the ovarian cancer exosomes was correlated with the mannan and mannan bead standard curves from Examples 2 and 3; mannan coated beads are approximately the same size as the ovarian cancer exosomes. Results from such experiments are shown in FIG. 3 and FIG. 4.

### Example Quantifying tuberculosis bacillus (TB) infected macrophage exosomes

A purified exosome sample from tuberculosis bacillus (TB) infected macrophages is incubated by the same method and on the same plate as the mannan or mannan latex bead standard curves described in Example 1, 2 and 3. The signal generated from the TB infected macrophage derived exosomes is correlated with a known amount of mannan or known number of mannan coated beads that are of approximately the same size as the TB infected macrophage derived exosome.

### Example 6-Optimizing mannan-coated beads

The lectin ELLSA assay was optimized with different concentrations of coated GNA. In one experiment, 100 µl of GNA in dPBS was incubated for 1 hour at ambient temperature in the wells of a 96 well plate at the following concentrations: 10 µg/ml, 5 µg/ml, 2.5 µg/ml, and 1.25 µg/ml. The GNA coating solution was then removed from the plate, and the plate blocked with 300 µl of 1% BSA, 0.1% Tween 20 for 1 hour at ambient temperature. Either mannan or mannan coated beads were incubated on the plate for 1 hour at ambient temperature at 1000 ng/ml, 100 ng/ml, 10 ng/ml and 1 ng/ml for mannan and 1e11 beads/ml, 1e10 beads/ml, 1e9 beads/ml, and 1e8 beads/ml for mannan latex beads, including 2 blank wells (dPBS alone) per GNA coating concentration to determine sensitivity and background of the assay. Mannan or mannan beads were incubated for 1 hour at ambient temperature on the GNA coated plates. Wells were washed 1 x with 300 µl wash solution. 100 µl of HRP labeled GNA (1 µg/ml) was incubated with bound mannan or mannan beads for 1 hour at ambient temperature. Wells were washed 4 x with 300 µl wash solution, and 100 µl of TMB was added to each well for 30 minutes or until color developed in the blank wells. The TMB HRP reaction was stopped with 1 M sulfuric acid, and the resulting yellow color was measured in a plate reader at 450 nm wavelength. The results are shown in FIG.s 5A and 5B. Substrates coated with 10 µg/ml GNA provided optimal results in this experiment.

### Example 7-Quantifying colon cancer exosomes

A 96-well plate was prepared according to Example 1. Various dilutions of cell culture media supernatants from human primary colon cancer cells were obtained.

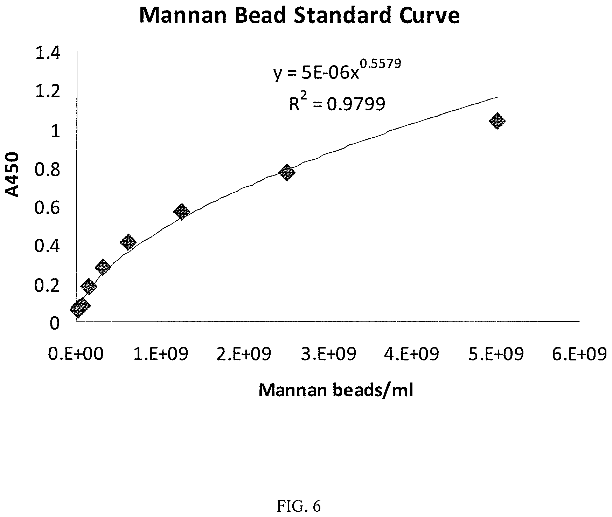

A 100 µl sample of a mannan bead standard or a supernatant sample was added to each well, and incubated for 1 hour at RT. The plates were washed 1 X with PBS + 0.1% Tween-20. Bound exosomes or bound mannan was detected by adding 100 µl 1 µg/ml GNA coupled to horse radish peroxidase (HRP), and incubating for 1 hour at RT, washing the plate 4 X with PBS + 0.1% Tween-20. HRP was detected and measured by adding 100 µl of tetramethylbenzidine, allowing the color to develop 30 minutes at RT, stopping the reaction with 100 µl 1 M sulfuric acid. Intensity of color was measured at an absorbance at 450 nm. FIG 6 shows the mannan bead standard curve. Table 1 summarizes the calculated exosome concentration relative to the mannan standard curve.

**TABLE 1**

| **Supernatant dilution** | **A₄₅₀** | **Exosome concentration relative to mannan bead standard curve(Beads/ml)** |
|---|---|---|
| 1.00 | 1.019 | 3.28E+09 |
| 0.1 | 0.08 | 3.43E+07 |
| 0.01 | 0.039 | 9.47E+06 |
| 0.001 | 0.028 | 5.23E+06 |

### Example 8-Quantifying melanoma exosomes

Melanoma exosome cell culture supernatants were prepared by plating out five melanoma cell lines (human primary melanoma cell lines). After five days, the cells were washed and then incubated for 48 hours in supplemented media. The supernatants were collected.

Exosomes were detected and measured as described in Example 7. Table 2 summarizes the Absorbance 450 nm measurements of diluted supernatants and the concentration of the exosomes relative to a mannan standard curve.

**TABLE 2**

| **Cell line** | **Supernatant dilution** | **A₄₅₀** | **Exosome concentration relative to mannan standard curve (ng/ml)** | **Average relative exosome concentration (ng/ml)** |
|---|---|---|---|---|
| CCS-1 | Undiluted | 0.298 | 24 | **25** |
| | 1:2 | 0.211 | 26 | |
| | 1:4 | 0.152 | 25 | |
| CCS-2 | Undiluted | 0.531 | 51 | **45** |
| | 1:2 | 0.267 | 40 | |
| | 1:4 | 0.191 | 43 | |
| CCS-3 | Undiluted | 0.171 | 8 | **17** |
| | 1:2 | 0.187 | 21 | |
| | 1:4 | 0.144 | 21 | |
| CCS-4 | Undiluted | 0.388 | 34 | **45** |
| | 1:2 | 0.313 | 51 | |
| | 1:4 | 0.203 | 49 | |
| CCS-5 | Undiluted | 0.263 | 19 | **20** |
| | 1:2 | 0.182 | 20 | |
| | 1:4 | 0.147 | 22 | |

### Example 9-Quantifying ovarian cancer exosomes

Various dilutions of human ascites fluid from a ovarian cancer patient were obtained. Exosomes in the fluid were quantified according to a method similar to the method described in Example 7. Table 3 summarizes the calculated concentrations of the ovarian exosomes relative to mannan.

**TABLE 3**

| **Supernatant dilution** | **A₄₅₀** | **Exosome concentration relative to mannan standard curve (ng/ml)** |
|---|---|---|
| 1 | 0.404 | 55.6 |
| 0.5 | 0.259 | 35.4 |
| 0.25 | 0.163 | 22.1 |
| 0.125 | 0.115 | 15.4 |
| 0.0625 | 0.073 | 9.6 |
| 0 | 0 | -0.5 |

### Example 10-Quantifying exosomes derived from tuberculosis infected macrophage

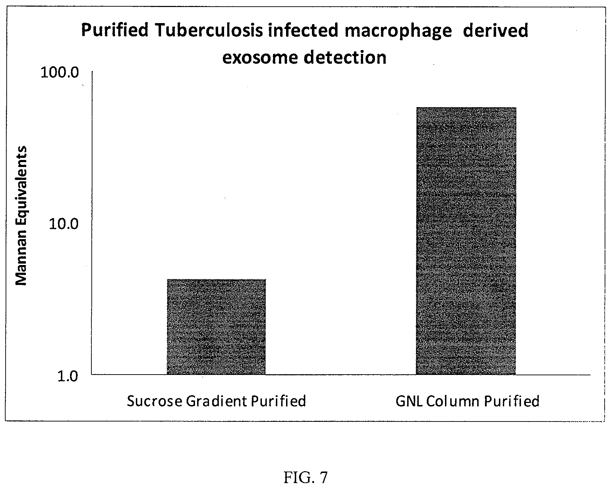

Exosomes from murine tuberculosis infected macrophage were purified by using a sucrose gradient, or using a GNA resin and eluting with 1 M alpha methyl-mannoside. Exosome concentrations were determined using a method similar to the method of Example 7. FIG. 7 summarizes the results.

### Example 11-Quantifying ovarian cancer exosomes with Anti-PLAP antibody

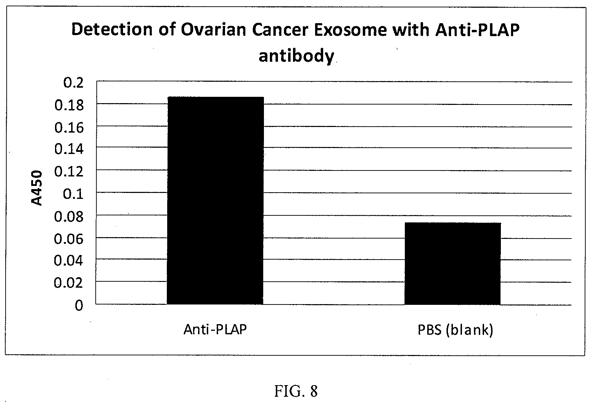

The ability of anti-placental alkaline phosphatase (PLAP) to detect ovarian cancer exosomes bound to GNA coated plates was tested. GNA coated multiwell plates were prepared as described in Example 1. Ovarian cancer cell exosomes were incubated in the wells. The presence of exosomes was determined using labeled anti-PLAP antibody which detects a marker of ovarian cells. The results are summarized in FIG. 8.

The term "comprising" as used herein is synonymous with "including," "containing," or "characterized by," and is inclusive or open-ended and does not exclude additional, unrecited elements or method steps.

## Claims

1. A method for quantifying exosomes in a sample comprising:
contacting the sample with lectin immobilized on a substrate;
contacting exosomes bound to the lectin with a detectable exosome-binding agent; and
measuring a signal from the bound detectable exosome-binding agent, thereby quantifying the bound exosomes;
wherein the lectin is selected from the group consisting of *Galanthus nivalis* lectin (GNA), *Narcissus pseudonarcissus* lectin (NPA), *Allium sativum* lectin (ASA), *Lens culinaris* lectin (LCH), *Sambucus nigra* lectin (SNA), *Maackia amurensis* lectin (MAL), and concanavalin A.

2. The method of claim 1, further comprising comparing the signal from the bound detectable binding agent to a signal on a standard curve.

3. The method of any one of claims 1-2, wherein the exosomes are derived from a cell selected from the group consisting of an ovarian cancer cell, a melanoma cell, a colon cancer cell, and a tuberculosis infected cell.

4. The method of any one of claims 1-3, wherein the method provides a sensitivity of detecting exosomes in the sample that is at least 1 x 10⁹ exosomes/ml, preferably at least 1 x 10⁸ exosomes/ml, or more preferably at least 1 x 10⁷ exosomes/ml.

5. The method of any one of claims 1-4, wherein the sample comprises an exosome having a diameter of 10 nm to 800 nm, or preferably a diameter of 30 nm to 200 nm.

6. The method of any one of claims 1-5, wherein the sample comprises exosomes isolated from a fluid by a method selected from the group consisting of size exclusion chromatography, density gradient centrifugation, differential centrifugation, nanomembrane ultrafiltration, immunoabsorbent capture, affinity purification, microfluidic separation, and a combination thereof.

7. The method of any one of claims 1-6, wherein at least one of the lectin immobilized on the substrate and the detectable binding agent are capable of binding to exosomes derived from a plurality of cell types.

8. The method of any one of claims 1-7, wherein the detectable exosome-binding agent is selected from the group consisting of an antibody, an antibody fragment, and a lectin selected from the group consisting of *Galanthus nivalis* lectin (GNA), *Narcissus pseudonarcissus* lectin (NPA), *Allium sativum* lectin (ASA), *Lens culinaris* lectin (LCH), *Sambucus nigra* lectin (SNA), *Maackia amurensis* lectin (MAL), and concanavalin A; and optionally wherein the detectable exosome-binding agent comprises a detectable label selected from the group consisting of an enzyme, a chemiluminescent agent, a fluorescent agent, and an isotope.

9. The method of any one of claims 1-8, wherein the sample is selected from the group consisting of peripheral blood, serum, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, umbilical cord blood, and ascites fluid.

10. The method of any one of claims 1-9, wherein the substrate comprises a multiwell plate comprising a material selected from the group consisting of sepharose, latex, glass, polystyrene, polyvinyl, nitrocellulose and silicon.

## Patentansprüche

1. Verfahren zur Quantifizierung von Exosomen in einer Probe, umfassend:
Kontaktieren der Probe mit auf einem Substrat immobilisierten Lectin;
Kontaktieren der an das Lectin gebundenen Exosome mit einem nachweisbaren Exosom-Bindemittel; und
Messen eines Signals von dem gebundenen nachweisbaren Exosom-Bindemittel zur Quantifizierung der gebundenen Exosome;
wobei das Lectin ausgewählt ist aus der Gruppe, bestehend aus Lectin aus *Galanthus nivalis* (GNA), Lectin aus *Narcissus pseudonarcissus* (NPA), Lectin aus *Allium sativum* (ASA), Lectin aus *Lens culinaris* (LCH), Lectin aus *Sambucus nigra* (SNA), Lectin aus *Maackia amurensis* (MAL) und Concanavalin A.

2. Verfahren nach Anspruch 1, weiterhin umfassend das Vergleichen des Signals des gebundenen nachweisbaren Bindemittels mit einem Signal auf einer Standardkurve.

3. Verfahren nach einem der Ansprüche 1-2, wobei die Exosome abgeleitet sind von einer Zelle, ausgewählt aus der Gruppe, bestehend aus einer Eierstockkrebszelle, einer Melanomzelle, einer Darmkrebszelle und einer mit Tuberkulose infizierten Zelle.

4. Verfahren nach einem der Ansprüche 1-3, wobei das Verfahren eine Empfindlichkeit des Nachweises von Exosomen in der Probe bereitstellt, die mindestens 1 x 10⁹ Exosome/ml, vorzugsweise mindestens 1 x 10⁸ Exosome/ml und mehr bevorzugt mindestens 1 x 10⁷ Exosome/ml beträgt.

5. Verfahren nach einem der Ansprüche 1-4, wobei die Probe ein Exosom mit einem Durchmesser von 10 nm bis 800 nm oder vorzugsweise einem Durchmesser von 30 nm bis 200 nm umfasst.

6. Verfahren nach einem der Ansprüche 1-5, wobei die Probe Exosome umfasst, die mittels eines Verfahrens, ausgewählt aus der Gruppe, bestehend aus Größenausschlusschromatografie, Dichtegradientenzentrifugation, Differenzialzentrifugation, Nanomembranultrafiltration, Immunoabsorbent-Capture, Affinitätsreinigung, mikrofluidischer Separation und einer Kombination davon aus einem Fluid isoliert sind.

7. Verfahren nach einem der Ansprüche 1-6, wobei mindestens entweder das auf dem Substrat immobilisierte Lectin oder das nachweisbare Bindemittel an Exosome binden kann, die aus einer Mehrzahl von Zelltypen abgeleitet sind.

8. Verfahren nach einem der Ansprüche 1-7, wobei das nachweisbare Exosom-Bindemittel ausgewählt ist aus der Gruppe, bestehend aus einem Antikörper, einem Antikörperfragment und einem Lectin, ausgewählt aus der Gruppe, bestehend aus Lectin aus *Galanthus nivalis* (GNA), Lectin aus *Narcissus pseudonarcissus* (NPA), Lectin aus *Allium sativum* (ASA), Lectin aus *Lens culinaris* (LCH), Lectin aus *Sambucus nigra* (SNA), Lectin aus *Maackia amurensis* (MAL) und Concanavalin A; und optional wobei das nachweisbare Exosom-Bindemittel einen nachweisbaren Marker umfasst, ausgewählt aus der Gruppe, bestehend aus einem Enzym, einem Chemilumineszenzmittel, einem Fluoreszenzmittel und einem Isotop.

9. Verfahren nach einem der Ansprüche 1-8, wobei die Probe ausgewählt ist aus der Gruppe, bestehend aus peripherem Blut, Serum, Plasma, Aszites, Urin, Rückenmarkflüssigkeit, Sputum, Speichel, Knochenmark, Synovialflüssigkeit, wässriger Körperflüssigkeit, Fruchtwasser, Cerumen, Muttermilch, broncheoalveolarer Spülflüssigkeit, Samen, Prostatasekret, Cowper-Sekret oder Präejakulat, weiblichem Ejakulat, Stuhl, Haar, Tränen, Zystensekret, Pleura- und Peritonealflüssigkeit, Perikardflüssigkeit, Lymphe, Chymus, Chylus, Galle, interstitieller Flüssigkeit, Menses, Eiter, Sebum, Vomitus, Scheidensekret, Schleimhautsekret, Stuhlwasser, Pankreassekret, Spülwasser der Nebenhöhlen, bronchopulmonalen Aspiraten, Blastocoelflüssigkeit, Nabelschnurblut und Aszitesflüssigkeit.

10. Verfahren nach einem der Ansprüche 1-9, wobei das Substrat einer Multiwell-Platte umfasst, umfassend ein Material, ausgewählt aus der Gruppe, bestehend aus Sepharose, Latex, Glas, Polystyrol, Polyvinyl, Nitrocellulose und Silikon.

## Revendications

1. Procédé de quantification d'exosomes dans un échantillon comprenant :
la mise en contact de l'échantillon avec une lectine immobilisée sur un substrat ;
la mise en contact d'exosomes liés à la lectine avec un agent de liaison
d'exosomes détectable ; et
la mesure d'un signal provenant de l'agent de liaison d'exosomes détectable
lié, et ainsi la quantification des exosomes liés ;
dans lequel la lectine est choisie dans le groupe consistant en : lectine de *Galanthus nivalis* (GNA), lectine de *Narcissus pseudonarcissus* (NPA), lectine d'*Allium sativum* (ASA), lectine de *Lens culinaris* (LCH), lectine de *Sambucus nigra* (SNA), lectine de *Maackia amurensis* (MAL) et concanavaline A.

2. Le procédé de la revendication 1, comprenant également la comparaison du signal provenant de l'agent de liaison détectable lié avec un signal sur une courbe standard.

3. Le procédé de l'une quelconque des revendications 1 à 2, dans lequel les exosomes sont issus d'une cellule choisie dans le groupe consistant en : une cellule de cancer de l'ovaire, une cellule de mélanome, une cellule de cancer du côlon et une cellule infectée par la tuberculose.

4. Le procédé de l'une quelconque des revendications 1 à 3, dans lequel le procédé possède une sensibilité de détection d'exosomes dans l'échantillon qui est au moins de 1 x 10⁹ exosomes/ml, préférablement au moins de 1 x 10⁸ exosomes/ml, ou plus préférablement au moins de 1 x 10⁷ exosomes/ml.

5. Le procédé de l'une quelconque des revendications 1 à 4, dans lequel l'échantillon comprend un exosome possédant un diamètre de 10 nm à 800 nm, ou préférablement un diamètre de 30 nm à 200 nm.

6. Le procédé de l'une quelconque des revendications 1 à 5, dans lequel l'échantillon comprend des exosomes isolés d'un liquide par une méthode choisie dans le groupe consistant en : chromatographie par exclusion, centrifugation en gradient de densité, centrifugation différentielle, ultrafiltration nano-membranaire, capture par immuno-absorption, purification par affinité, séparation microfluidique et une combinaison de ces méthodes.

7. Le procédé de l'une quelconque des revendications 1 à 6, dans lequel au moins la lectine immobilisée sur le substrat ou l'agent de liaison détectable est capable de se lier à des exosomes issus de plusieurs types de cellules.

8. Le procédé de l'une quelconque des revendications 1 à 7, dans lequel l'agent de liaison d'exosomes détectable est choisi dans le groupe consistant en : un anticorps, un fragment d'anticorps et une lectine choisie dans le groupe consistant en : lectine de *Galanthus nivalis* (GNA), lectine de *Narcissus pseudonarcissus* (NPA), lectine *d'Allium sativum* (ASA), lectine de *Lens culinaris* (LCH), lectine de *Sambucus nigra* (SNA), lectine de *Maackia amurensis* (MAL) et concanavaline A ; et éventuellement dans lequel l'agent de liaison d'exosomes détectable comprend un marqueur choisi dans le groupe consistant en : une enzyme, un agent chimioluminescent, un agent fluorescent et un isotope.

9. Le procédé de l'une quelconque des revendications 1 à 8, dans lequel l'échantillon est choisi dans le groupe consistant en : sang périphérique, sérum, plasma, ascite, urine, liquide céphalo-rachidien (LCR), expectoration, salive, moelle osseuse, liquide synovial, humeur aqueuse, liquide amniotique, cérumen, lait maternel, liquide de lavage broncho-alvéolaire, sperme, liquide prostatique, liquide de Cowper ou liquide pré-éjaculatoire, éjaculat féminin, sueur, matière fécale, cheveu, larmes, liquide de kyste, liquide pleural et péritonéal, liquide péricardique, lymphe, chyme, chyle, bile, liquide interstitiel, menstrues, pus, sébum, vomi, sécrétions vaginales, sécrétions des muqueuses, eau fécale, suc pancréatique, liquide de lavage des sinus, aspirations broncho-pulmonaires, liquide de blastocèle, sang de cordon ombilical et liquide d'ascite.

10. Le procédé de l'une quelconque des revendications 1 à 9, dans lequel le substrat comprend une plaque multipuits comprenant une matière choisie dans le groupe consistant en Sepharose, latex, verre, polystyrène, polyvinyle, nitrocellulose et silicone.
